# EUROPEAN PATENT SPECIFICATION

(11) **EP 4 050 335 B1**
(45) Date of publication and mention of the grant of the patent: **16.10.2024**
(21) Application number: 19950908.4
(22) Date of filing: 13.12.2019
(51) Int. Cl.: G01N 33/487, G01N 33/49, G01N 27/00, A61B 5/15, A61B 5/151, A61B 5/155, A61B 5/157, G01N 27/327

(54) **BLOOD GLUCOSE MONITORING DEVICE AND SYSTEM**
BLUTZUCKERÜBERWACHUNGSVORRICHTUNG UND -SYSTEM
DISPOSITIF ET SYSTÈME DE SURVEILLANCE DE LA GLYCÉMIE

(30) Priority: 29.10.2019 CN 201911039959; 29.10.2019 CN 201921849294 U
(43) Date of publication of application: 31.08.2022
(73) Proprietor: Zhejiang Summed Medtech Co., Ltd., Huzhou, Zhejiang 313000 (CN)
(72) Inventor: WENG, Jiansen, Shanghai 200050 (CN); LI, Dehui, Shanghai 200050 (CN)
(74) Representative: Schwerbrock, Florian
(86) International application number: PCT/CN2019/125232
(87) International publication number: WO 2021/082208

(56) References cited:
- EP-A1- 2 799 004
- EP-A2- 2 446 819
- WO-A2-2007/064596
- WO-A2-2012/028675
- CN-A- 101 036 053
- CN-A- 104 330 444
- CN-A- 104 330 444
- CN-A- 104 634 821
- CN-U- 206 499 468
- US-A1- 2003 199 902
- US-A1- 2014 323 915
- US-A1- 2017 110 781

## Description

### FIELD OF DISCLOSURE

The present invention relates to a blood glucose detection device according to the preamble of claim 1. Such a blood glucose detection device is known from WO 2012/028675 A2. The present invention also relates to a blood glucose detection device according to the preamble of claim 8. Such a blood glucose detection device is known from EP 2 446 819 A2. The present invention also relates to a blood glucose detection system.

### BACKGROUND OF THE INVENTION

The current equipment that tests blood glucose is mainly a blood glucose detection device, but it is inconvenient to carry when the patient goes out, and is also relatively cumbersome during use as a new test strip needs to be replaced every time a test is performed. Besides, it is difficult to transmit data in the blood glucose detection device externally for management. Therefore, it is necessary to provide a portable blood glucose detection device, making it convenient for patients to use when they go out.

### SUMMARY OF INVENTION

One object of the application is to provide a kind of blood glucose detection device and system, for avoiding the problem that existing blood glucose detection device is inconvenient to carry and blood glucose test strips needs to be replaced when using.

In order to achieve the above object, the present invention provides a blood glucose detection device according to claim 1. Preferred embodiments of the present invention are defined in the dependent claims.

Furthermore, there are a plurality of lancing units. The amount is between 2 and 20.

Furthermore, these lancing units are provided with intervals between two, and are provided along the edge of the portable card-type base.

Furthermore, these lancing units are also covered with a protective film, which is equipped with a siphon groove. And the opening of the siphon groove is located at the edge of the portable card-type base and communicated with the outside.

The test unit further includes a communication module and a current detection module, and the communication module is electrically connected to the current detection module.

Furthermore, the communication module is a near field communication chip.

The device further includes a temperature detection unit, which is equipped on the portable card-type base, and is electrically connected with the communication module.

According to the present invention , there is provided a blood glucose detection device according to claim 8. Preferred embodiments of the present invention are defined in the dependent claims.

The device further includes a lancet protection unit. The lancet is connected to the portable housing through a spring, and the lancet protection unit is embedded in the cavity of the portable housing, and is set at the front end of the lancet.

The lancet protection unit further comprises a protective head and a protective sleeve. The front end of the protective sleeve is provided with through-hole and the rear end of the protective sleeve is embedded in the cavity. The lancet can extend through the through-hole and the protection head is used to block the through-hole.

Furthermore, there are a plurality of lancing units. The amount is between 1 and 4.

Furthermore, these lancing units are provided with intervals between two, and are provided along the cavity opening of the portable housing.

Furthermore, the lancing unit is also covered with a protective film, which is equipped with a siphon groove. And the opening of the siphon groove is located at the cavity opening of the portable housing and communicated with the outside.

The test unit further includes a communication module and a current detection module, and the communication module is electrically connected to the current detection module

Furthermore, the communication module is a near field communication chip.

The device further includes a temperature detection unit, which is equipped on the portable housing, and is electrically connected with the communication module.

According to the present invention, there is also provided a blood glucose detection system according to claim 17. Preferred embodiments are defined in the dependent claims.

The mobile terminal, further comprising: a near field communication reading device, which is used to obtain the current intensity sent by the blood glucose detection device.

The blood glucose detection device further comprises a temperature detection unit and the mobile terminal is also used for receiving the temperature that the temperature detection unit obtains. If the temperature is in preset temperature range, then it determines the blood glucose concentration according to the current intensity.

Compared with the prior art, the application presents a blood glucose detection device and system, which can provide a portable blood glucose detection device for patients. The blood glucose detection device may include a plurality of lancing units, which collect patient blood and generate current. The current is detected by the test unit and can transmit the current intensity data to the external device, thus making the glucose detection device portable. Besides, there is no need to change test strips every time one performs a blood glucose test as the current intensity data can be transmitted externally for management, which improves the patient's experience and makes it convenient for patients to perform blood glucose tests when they go out.

### BRIEF DESCRIPTION OF THE DRAWINGS

Other features, objects and advantages of the present application will become more apparent with the detailed description of the non-limiting embodiments made with reference to the following drawings:
FIG. 1 is the structural diagram of the embodiment of the blood glucose detection device;
FIG. 2 is the siphon groove structural diagram of the embodiment of the blood glucose detection device;
FIG. 3 is the circuit structural diagram of the embodiment of the blood glucose detection device;
FIG. 4 is the structural diagram of the embodiment of the preferred blood glucose detection device;
FIG. 5 is the structural diagram of the other embodiment of the blood glucose detection device;
FIG. 6 is the structural diagram of the other embodiment of the blood glucose detection device;
FIG. 7 is the siphon groove structural diagram of the other embodiment of the blood glucose detection device;
FIG. 8 is the circuit structural diagram of the other embodiment of the blood glucose detection device.

Description of the drawing elements: 1. portable card-type base; 2. lancing unit; 3. test unit; 4. groove; 5. protective film; 6. siphon groove; 7. communication module; 8. current detection module; 9. temperature detection unit; 10. portable housing; 11. lancet; 12. protective head; 13. protective sleeve.

The same or similar reference signs in the drawings represent the same or similar elements.

### DETAILED DESCRIPTION

The application will be described in further detail below in conjunction with the accompanying drawings.

In a typical configuration of the application, the terminal and the network device each include one or more processors (CPU), an input/output interface, a network interface and a memory.

The memory may include non-persistent memory, random access memory (RAM) and/or non-volatile memory in computer readable media, such as, read only memory (ROM) or flash memory (flash RAM). Memory is an example of a computer-readable medium.

Computer-readable media, including persistent and non-persistent, removable and non-removable media, can achieve information storage by any method or technology. The information may be computer readable instructions, data structures, modules of programs, or other data. Examples of computer storage media include, but are not limited to, phase-change random access memory (PRAM), static random access memory (SRAM), dynamic random access memory (DRAM), other types of random access memory (RAM), read only memory (ROM), electrically erasable programmable read only memory (EEPROM), flash memory or other memory technology, compact disc read only memory (CD-ROM), digital versatile disc (DVD) or other optical storage, magnetic tape cartridges, magnetic tape disk storage or other magnetic storage devices or any other non-transmission medium, that can be used to store information that can be accessed by a computing device. Computer-readable media, as defined herein, excludes non-transitory computer-readable media, such as modulated data signals and carrier waves.

The application provides a blood glucose detection device, wherein, the blood glucose detection device comprises a portable card-type base 1, at least one lancing unit 2 and a test unit 3. At least one lancing unit 2 is arranged on the edge of the portable card-type base 1. The lancing unit 2 includes a groove 4 provided on the portable card-type base 1. The groove 4 is coated with glucose oxidase or glucose dehydrogenase and the lancing unit 2 is used to collect blood and generate electric current. The test unit 3 is arranged on the portable card-type base 1 and electrically connected with the lancing unit 2, and is used for detecting the intensity of the generated current and communicating with the outside.

Wherein, the size and weight of the portable card-type base 1 are similar to bank cards, transportation cards, etc. commonly used in daily life, which are convenient for patients to carry when they go out, and can be placed in similar wallets and carry-on bags.

The lancing unit 2 is arranged on the portable card-type base 1, one end of which is arranged at the edge of the portable card-type base 1, and the other end is arranged in the middle area of the portable card-type base 1, and is used for collecting the blood of the patient and generating electricity according to the chemical reactions of blood flow. In order to facilitate the collection of the patient's blood, the embodiment of the application is also provided with a groove 4 for collecting blood on the portable card-type base 1. The groove 4 is set near the edge of the portable card base 1, and the inner wall of the groove 4 is also coated with glucose oxidase or glucose dehydrogenase for reacting with glucose in blood to generate electric current. The blood of the patient can be fully collected by the groove 4 to avoid blood dispersion, so that the blood can fully react with the glucose oxidase or glucose dehydrogenase in the groove 4 to avoid the error of blood glucose detection caused by insufficient reaction and improve the accuracy of blood glucose detection.

In the embodiment of the application, in order to reduce the trivial operation that the patient needs to replace the test strip when performing blood glucose detection each time, a plurality of disposable lancing units 2 are provided on the portable card-type base 1, and the specific number of lancing units 2 can be set according to the size and convenience of use of the portable card-based base 1. Preferably, the number can be set between 2 and 20.

In addition, in order to avoid blood contamination between the lancing units 2, a certain interval is set between each two lancing units 2, and the specific interval distance can be determined according to actual use requirements. The lancing units 2 can be arranged in sequence along the edge of the portable card-type base 1, and two groups of lancing units 2 can be simultaneously arranged on the two longer sides of the portable card-type base 1.

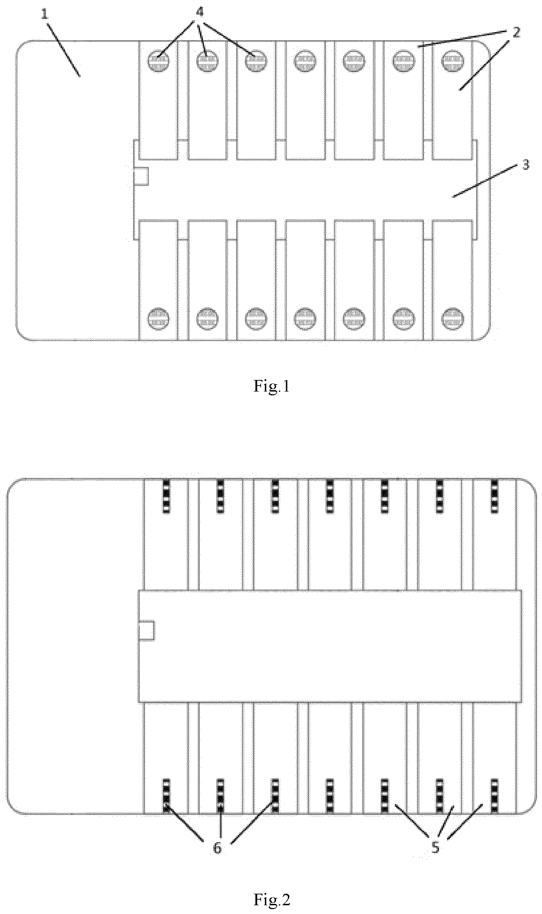

In the embodiment of the application, the lancing unit 2 is also covered with a protective film 5. As shown in FIG. 2, the protective film 5 can be used to protect the glucose oxidase or glucose dehydrogenase in the lancing unit from being subjected to external pollution or influence, thereby extending the service life and improving the accuracy of test. In addition, the protective film 5 is also provided with a siphon groove 6. The opening of the siphon groove 6 is at the edge of the portable card-type base 1 and communicates with the outside. The other end of the siphon groove 6 extends to the groove 4 in the lancing unit 2. The siphon groove 6 has a siphoning action that siphons the patient's blood located in the opening to the groove 4, thus eliminating the need for the patient to drip blood into the groove 4, facilitating the patient's blood collection operation while avoiding possible contamination near the groove 4 when dripping blood.

In addition, the lancing unit 2 is also electrically connected with the test unit 3 provided on the portable card-type base 1, and the lancing unit 2 transmits the generated current to the test unit 3. Specifically, the groove 4 in the lancing unit 2 is electrically connected to the test unit 3, and the current generated by the groove 4 is transmitted to the test unit 3 for current intensity detection through the current. The test unit 3 can transmit the current intensity data to an external device in real time, or can temporarily store the current intensity data and delay the transmission, or provide the corresponding current intensity data according to the data acquisition request of the external device.

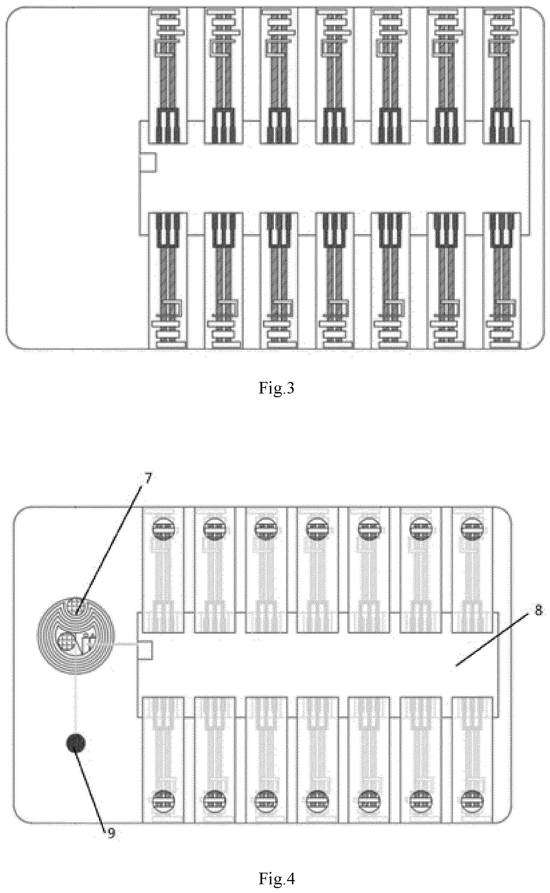

Wherein, the connection circuit between the lancing unit 2 and the test unit 3 adopts the three-electrode system in electrochemical analysis, as shown in FIG. 3. Compared with the traditional two-electrode system, the three-electrode system includes working electrode, reference electrode and counter electrode. The reference electrode is used to set the zero point. The current flows through the working electrode and the reference electrode to form a system that is not or essentially less energized. The stability of the potential of the reference electrode is used to measure the electrode potential of the working electrode. The working electrode and the auxiliary electrode form an energized system that is used to measure the current flowing through the working electrode. The three-electrode measurement system is used to study the relationship between the point position of the working electrode and the current.

In the embodiment of the application, the test unit 3 may include a communication module 7 and a current detection module 8. As shown in FIG. 4, the communication module 7 is electrically connected to the current detection module 8. In addition, the lancing unit 2 is electrically connected to the current detection module 8, and the current generated by the lancing unit 2 is input to the current detection module 8 for current intensity detection. Preferably, the current detection module 8 may be a current detection chip, which receives the current signal output by the lancing unit 2 through the two electrodes via the three electrodes.

Preferably, the communication module 7 is a near-field communication chip, and near-field communication (NFC) is a short-distance high-frequency wireless communication technology. "Near field" refers to the proximity of electromagnetic field radio waves, which can realize exchange data in close proximity to each other. It evolved from the integration of contactless radio frequency identification (RFID) and interconnection and interoperability technology, which can integrate the functions of inductive reader, inductive card and peer-to-peer communication on a single chip.

In the embodiment of the present application, the lancing unit also includes a temperature detection unit 9, which is equipped on the portable card-type base 1, and is electrically connected with the communication module 7. Wherein, since blood glucose needs to be tested within a certain temperature range to achieve the highest accuracy. For example, the most accurate test is between 18 degrees Celsius and 25 degrees Celsius. Therefore, the temperature during blood sugar detection is obtained through the temperature detection unit 9, which can be used for judging whether the obtained blood glucose test results are meaningful.

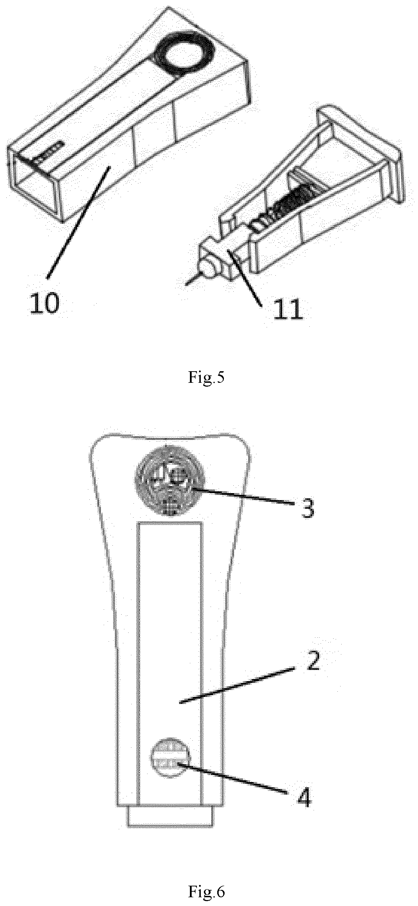

The other embodiment of the application also provide a blood glucose detection device. As shown in FIG.5 and 6, the blood glucose detection device comprises a portable housing 10 with cavity, lancet 11, at least one lancing unit 2 and a test unit 3. At least one lancing unit 2 is arranged on the cavity opening side of the portable housing 10. The lancing unit 2 includes a groove 4 provided on the portable housing 10. The groove 4 is coated with glucose oxidase or glucose dehydrogenase and the lancing unit 2 is used to collect blood and generate electric current. The test unit 3 is arranged on the portable housing 10 and electrically connected with the lancing unit 2, and is used for detecting the intensity of the generated current and communicating with the outside. The lancet 11 is arranged in the cavity of the portable housing 10.

Wherein, the lancet 11 is integrated into the blood glucose detection device, realizing the integration of the patient's blood acquisition and blood test, which is convenient for the patient to use, and avoids the inconvenience caused by the separation of the lancet and the blood glucose detection device.

The lancing unit 2 is arranged on the portable housing 10, one end of which is arranged at one side of the cavity opening of portable housing 10, and the other end is arranged at one side away from the cavity opening of the portable housing 10, and is used for collecting the blood of the patient and generating electricity according to the chemical reactions of blood flow. In order to facilitate the collection of the patient's blood, some embodiments of the application are also provided with a groove 4 for collecting blood on the portable housing 10. The groove 4 is set near the edge of the portable housing 10, and the inner wall of the groove 4 is also coated with glucose oxidase or glucose dehydrogenase for reacting with glucose in blood to generate electric current. The blood of the patient can be fully collected by the groove 4 to avoid blood dispersion, so that the blood can fully react with the glucose oxidase or glucose dehydrogenase in the groove 4 to avoid the error of blood glucose detection caused by insufficient reaction and improve the accuracy of blood glucose detection.

Preferably, in order to avoid the lancet 11 accidentally injuring the patient when blood collection is not required, the device also includes a lancet protection unit. Wherein, the lancet 11 is connected to the portable housing 10 through a spring, and the lancet protection unit is embedded in the cavity of the portable housing 10, and is set at the front end of the lancet 11.

Specifically, the lancet protection unit includes a protective head 12 and a protective sleeve 13. The front end of the protective sleeve 13 is provided with a through-hole while the rear end is embedded in the cavity of the portable housing 10. And the lancet 11 can extend through the through-hole. One end of the protective head 12 is provided with a protrusion, which is made of soft material and can be used to block the through-hole at the front of the protective sleeve 13. And lancet 11 can be pierced into the protrusion to prevent the front tip of lancet 11 from stabbing the patient.

In the embodiment of the application, in order to reduce the trivial operation that the patient needs to replace the test strip when performing blood glucose detection each time, a plurality of disposable lancing units 2 are provided on the outer wall of portable housing 10, and the specific number of lancing units 2 can be set according to the size and convenience of use of the portable housing 10. Preferably, the number can be set between 1 and 4.

In addition, in order to avoid blood contamination between the lancing units 2, a certain interval is set between each two lancing units 2, and the specific interval distance can be determined according to actual use requirements. The lancing units 2 can be arranged around the cavity opening of the portable housing 10.

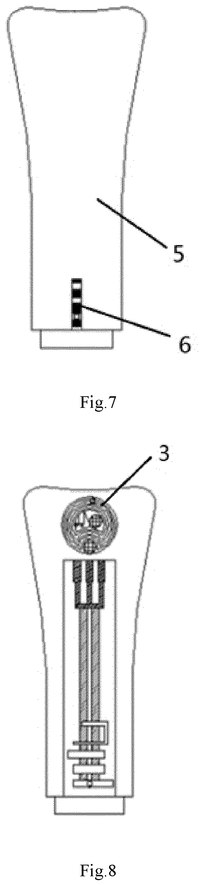

In the embodiment of the application, the lancing unit 2 is also covered with a protective film 5. As shown in FIG. 7, the protective film 5 can be used to protect the glucose oxidase or glucose dehydrogenase in the lancing unit from being subjected to external pollution or influence, thereby extending the service life and improving the accuracy of test. In addition, the protective film 5 is also provided with a siphon groove 6. The opening of the siphon groove 6 is at the cavity opening of the portable housing 10 and communicates with the outside. The other end of the siphon groove 6 extends to the groove 4 in the lancing unit 2. The siphon groove 6 has a siphoning action that siphons the patient's blood located in the opening to the groove 4, thus eliminating the need for the patient to drip blood into the groove 4, facilitating the patient's blood collection operation while avoiding possible contamination near the groove 4 when dripping blood.

In addition, the lancing unit 2 is also connected with the test unit 3 provided on the portable housing 10 through a circuit, and the lancing unit 2 transmits the generated current to the test unit 3. Specifically, the groove 4 in the lancing unit 2 is connected to the test unit 3 through a circuit, and the current generated by the groove 4 is transmitted to the test unit 3 for current intensity detection through the current. The test unit 3 can transmit the current intensity data to an external device in real time, or can temporarily store the current intensity data and delay the transmission, or provide the corresponding current intensity data according to the data acquisition request of the external device.

Wherein, the connection circuit between the lancing unit 2 and the test unit 3 adopts the three-electrode system in electrochemical analysis, as shown in FIG. 8.

In the embodiment of the application, the test unit 3 may include a communication module 7 and a current detection module 8 (not shown in the figure). The communication module 7 is electrically connected to the current detection module 8. In addition, the lancing unit 2 is electrically connected to the current detection module 8, and the current generated by the lancing unit 2 is input to the current detection module 8 for current intensity detection. Preferably, the current detection module 8 may be a current detection chip, which receives the current signal output by the lancing unit 2 through the two electrodes via the three electrodes.

Preferably, the communication module 7 is a near field communication chip.

In some embodiments of the present application, the lancing unit also includes a temperature detection unit 9 (not shown in the figure), which is equipped on the portable housing 10, and is electrically connected with the communication module 7.

According to another aspect of the application, the application also provides a blood glucose detection system. The blood glucose detection system includes any one of the aforementioned two blood glucose detection devices and a mobile terminal. The mobile terminal is equipped to receive the current intensity sent by the blood glucose detection devices and determine the blood glucose concentration according to the current intensity.

Specifically, the mobile terminal includes a near-field communication reading device, which can be used to obtain the current intensity sent by the glucose detection device. After the mobile terminal obtains the current intensity data, it then calculates according to the formula corresponding to the current intensity and glucose concentration to obtain the corresponding glucose concentration data, i.e., blood glucose data. Preferably, a corresponding software application can be installed on the mobile terminal to calculate the blood glucose data.

In the embodiment of the application, the blood glucose detection device also comprises a temperature detection unit and the mobile terminal is also used for receiving the temperature that the temperature detection unit obtains. If the temperature is in preset temperature range, then it determines the blood glucose concentration according to the current intensity. Wherein, the mobile terminal can determine whether the obtained current intensity data is meaningful according to the obtained temperature. If it is within the preset temperature range, then the test is considered normal and the obtained current intensity data is valid. Then the current intensity data can be used to determine the blood glucose. Otherwise, the test is considered abnormal, and the obtained current intensity data is a problematic data. At this time, the mobile terminal can remind the patient that there is a problem with the blood glucose test, and a re-test may be needed.

In conclusion, the application provides presents a blood glucose detection device and system, which can provide a portable blood glucose detection device for patients. The blood glucose detection device may include a plurality of lancing units, which collect patient blood and generate current. The current is detected by the test unit and can transmit the current intensity data to the external device, thus making the glucose detection device portable. Besides, there is no need to change test strips every time one performs a blood glucose test as the current intensity data can be transmitted externally for management, which improves the patient's experience and makes it convenient for patients to perform blood glucose tests when they go out.

It should be noted that the application may be implemented in software and/or a combination of software and hardware, such as an application specific integrated circuit (ASIC), a general-purpose computer, or any other similar hardware device. In one embodiment, the software program of the application may be executed by a processor to implement the s teps or functions described above. Likewise, the software programs of the application (including associated data structures) may be stored on a computer-readable recording medium, such as an RAM memory, magnetic or optical drives or floppy disks, and the like. In addition, some steps or functions of the application may be implemented in hardware. For example, a circuit may cooperate with a processor to perform various steps or functions.

In addition, a part of the application can be applied as a computer program product. Take a computer program instruction as an example. When it is executed by a computer, the method and/or technical solution according to the application can be invoked or provided by the operation of that computer. The program instructions that invoke the methods of the application may be stored in fixed or removable recording media, and/or transmitted via data streams in broadcast or other signal-bearing media, and/or stored in the working memory of the computer device on which the program instructions are executed. Wherein, an embodiment according to the application includes an apparatus. The apparatus includes a memory for storing computer program instructions and a processor for executing the program instructions, wherein: when the computer program instructions are executed by the processor, the apparatus is triggered to run the method and/or technical solution based on the preceding multiple embodiments according to the application.

## Claims

1. A blood glucose detection device, includes a portable card-type base (1), at least one lancing unit (2), wherein the lancing unit (2) is arranged on the edge of the portable card-type base (1) and is used to collect blood and generate electric current and a test unit (3) arranged on the portable card-type base (1) and electrically connected with the lancing unit (2) which is used for detecting the intensity of the generated current and communicating with the outside, **characterized in that**:
the lancing unit (2) includes a groove (4) provided on the portable card-type base (1) and a temperature detection unit (9) is equipped on the portable card-type base (2), wherein the groove (4) is coated with glucose oxidase or glucose dehydrogenase and the temperature detection unit (9) is to provide a certain temperature range when the blood glucose needs to be tested.

2. The blood glucose detection device of claim 1, wherein the number of the lancing unit (2) is between 2 and 20.

3. The blood glucose detection device of claim 2, wherein these lancing units (2) are provided with intervals between two, and are provided along the edge of the portable card-type base (1).

4. The blood glucose detection device of claim 1, wherein the lancing unit (2) is covered with a protective film (5), which is equipped with a siphon groove (6), and the opening of the siphon groove (6) is located at the edge of the portable card-type base (1) and communicated with the outside.

5. The blood glucose detection device of claim 1, wherein the test unit (3) comprises a communication module (7) and a current detection module (8), and the communication module (7) is electrically connected to the current detection module (8).

6. The blood glucose detection device of claim 5, wherein the communication module (7) is a near field communication chip.

7. The blood glucose detection device of claim 5, wherein the lancing unit (2) is electrically connected with the communication module (7).

8. A blood glucose detection device, includes a portable housing (10) with cavity, a lancet (11) arranged in a cavity of a portable housing (10), at least one lancing unit (2) , wherein the lancing unit is arranged on the cavity opening side of the portable housing and is used to collect blood and generate electric current,
and a test unit (3) arranged on the portable housing (10) and electrically connected with the lancing unit (2), and used for detecting the intensity of the generated current and communicating with the outside, **characterized in that** the lancing unit (2) includes a groove (4) provided on the portable housing (10) and a temperature detection unit (9) is equipped on the portable card-type base (2), wherein the groove (4) is coated with glucose oxidase or glucose dehydrogenase and the temperature detection unit (9) is to provide a certain temperature range when the blood glucose needs to be tested.

9. The blood glucose detection device of claim 8, further comprising a lancet protection unit, the lancet (11) is connected to the portable housing (10) through a spring, and the lancet protection unit is embedded in the cavity of the portable housing (10), and is set at the front end of the lancet (11).

10. The blood glucose detection device of claim 9, wherein the lancet protection unit comprises a protective head (12) and a protective sleeve (13), the front end of the protective sleeve (13) is provided with through-hole and the rear end of the protective sleeve (13) is embedded in the cavity, the lancet (11) can extend through the through-hole and the protection head (12) is used to block the through-hole.

11. The blood glucose detection device of claim 8, wherein the_number of the lancing unit (2) is between 1 and 4.

12. The blood glucose detection device of claim 9, wherein these lancing units (2) are provided with intervals between two, and are provided along the cavity opening of the portable housing (10).

13. The blood glucose detection device of claim 8, wherein the lancing unit (2) is covered with a protective film (5), which is equipped with a siphon groove (6), and the opening of the siphon groove (6) is located at the cavity opening of the portable housing (10) and communicated with the outside.

14. The blood glucose detection device of claim 8, wherein the test unit (3) comprises a communication module (7) and a current detection module (8), and the communication module (7) is electrically connected to the current detection module (8).

15. The blood glucose detection device of claim 14, wherein the communication module (7) is a near field communication chip.

16. The blood glucose detection device of claim 14, wherein the lancing unit (2) is electrically connected with the communication module (7).

17. A blood glucose detection system comprising the blood glucose detection device according to any one of claims 1 to 7 or the blood glucose detection device according to any one of claims 8 to 16 and a mobile terminal, wherein the mobile terminal is equipped to receive the current intensity sent by the blood glucose detection device and determine the blood glucose concentration according to the current intensity.

18. The blood glucose detection system of claim 17, wherein the mobile terminal comprises a near field communication reading device, which is used to obtain the current intensity sent by the blood glucose detection device.

19. The blood glucose detection system of claim 17, wherein the blood glucose detection device further comprises a temperature detection unit (9) and the mobile terminal is used for receiving the temperature that the temperature detection unit (9) obtains, if the temperature is in preset temperature range, it determines the blood glucose concentration according to the current intensity.

## Patentansprüche

1. Das Blutzuckermessgerät, das eine mobile kartenartige Basis (1), mindestens eine Stecheinheit (2), wobei die Stecheinheit (2) am Rand der mobilen kartenartigen Basis (1) angeordnet ist und zum Sammeln von Blut und zum Erzeugen von elektrischem Strom verwendet wird, und eine Testeinheit (3) umfasst, die auf der mobilen kartenartigen Basis (1) angeordnet und elektrisch mit der Stecheinheit (2) verbunden ist, die zum Erfassen der Intensität des erzeugten Stroms und zur Kommunikation mit der Außenwelt verwendet wird, **dadurch gekennzeichnet, dass**:
die Stecheinheit (2) eine Rille (4) umfasst, die auf der mobilen kartenartigen Basis (1) vorgesehen ist, und eine Temperaturmesseinheit (9) auf der mobilen kartenartigen Basis (2) ausgestattet ist, wobei die Nut (4) mit Glukoseoxidase oder Glukosedehydrogenase beschichtet ist und die Temperaturmesseinheit (9) dazu dient, einen bestimmten Temperaturbereich bereitzustellen, wenn der Blutzucker getestet werden muss.

2. Blutzuckermessgerät gemäß der Anspruch 1, wobei die Anzahl der Stecheinheiten (2) zwischen 2 und 20 liegt.

3. Blutzuckermessgerät gemäß der Anspruch 2, wobei diese Stecheinheiten (2) in Abständen von zwei Stück und entlang des Randes der mobilen kartenartigen Basis (1) vorgesehen sind.

4. Blutzuckermessgerät gemäß der Anspruch 1, wobei die Stecheinheit (2) mit einer Schutzfolie (5) bedeckt ist, die mit einer Siphonrille (6) ausgestattet ist, und die Öffnung der Siphonrille (6) sich am Rand der mobilen kartenartigen Basis (1) befindet und mit der Außenseite verbunden ist.

5. Blutzuckermessgerät gemäß der Anspruch 1, wobei die Testeinheit (3) ein Kommunikationsmodul (7) und ein Stromerkennungsmodul (8) umfasst, und das Kommunikationsmodul (7) elektrisch mit dem Stromerkennungsmodul (8) verbunden ist.

6. Blutzuckermessgerät gemäß der Anspruch 5, wobei das Kommunikationsmodul (7) ein Nahfeldkommunikationschip ist.

7. Blutzuckermessgerät gemäß der Anspruch 5, wobei die Stecheinheit (2) elektrisch mit dem Kommunikationsmodul (7) verbunden ist.

8. Blutzuckermessgerät, bestehend aus einem mobilen Gehäuse (10) mit einem Hohlraum, einer Lanzette (11), die in einem Hohlraum eines mobilen Gehäuses (10) angeordnet ist, mindestens eine Stecheinheit (2), wobei die Stecheinheit (2) an der Hohlraumöffnungsseite des mobilen Gehäuses angeordnet ist und verwendet wird, um Blut zu sammeln und elektrischen Strom zu erzeugen, und eine Testeinheit (3) umfasst, die an dem mobilen Gehäuse (10) angeordnet ist und elektrisch mit der Stecheinheit (2) verbunden ist und verwendet wird, um die Intensität des erzeugten Stroms zu erfassen und mit der Umgebung zu kommunizieren, **dadurch gekennzeichnet, dass**:
die Stecheinheit (2) eine Rille (4) aufweist, die an dem mobilen Gehäuse (10) vorgesehen ist, und mit einer Temperaturmesseinheit (9) an der mobilen kartenartigen Basis (2) ausgestattet ist, wobei die Rille (4) mit Glukoseoxidase oder Glukosedehydrogenase beschichtet ist und die Temperaturmesseinheit (9) dazu dient, einen bestimmten Temperaturbereich bereitzustellen, wenn der Blutzucker getestet werden muss.

9. Blutzuckermessgerät gemäß der Anspruch 8, ferner umfassend: eine Lanzettenschutzeinheit, wobei die Lanzette (11) über eine Feder mit dem mobilen Gehäuse (10) verbunden ist und die Lanzettenschutzeinheit in den Hohlraum des mobilen Gehäuses (10) eingebettet ist und am vorderen Ende der Lanzette (11) angebracht ist.

10. Blutzuckermessgerät gemäß der Anspruch 9, wobei die Lanzettenschutzeinheit einen Schutzkopf (12) und eine Schutzhülse (13) umfasst, das vordere Ende der Schutzhülse (13) mit einem Durchgangsloch versehen ist und das hintere Ende der Schutzhülse (13) in den Hohlraum eingebettet ist, die Lanzette (11) sich durch das Durchgangsloch erstrecken kann und der Schutzkopf (12) zum Blockieren des Durchgangslochs verwendet wird.

11. Blutzuckermessgerät gemäß der Anspruch 8, wobei die Anzahl der Stecheinheiten (2) zwischen 1 und 4 beträgt.

12. Blutzuckermessgerät gemäß der Anspruch 9, wobei diese Stecheinheiten (2) in Intervallen von zwei vorgesehen sind und entlang der Hohlraumöffnung des mobilen Gehäuses (10) angeordnet sind.

13. Blutzuckermessgerät gemäß der Anspruch 8, wobei die Stecheinheit (2) mit einer Schutzfolie (5) überzogen ist, die mit einer Siphonrille (6) versehen ist, und sich die Öffnung der Siphonrille (6) an der Hohlraumöffnung des mobilen Gehäuses (10) befindet und mit der Außenwelt verbunden ist.

14. Blutzuckermessgerät gemäß der Anspruch 8, wobei die Testeinheit (3) ein Kommunikationsmodul (7) und ein Stromerkennungsmodul (8) umfasst, und das Kommunikationsmodul (7) elektrisch mit dem Stromerkennungsmodul (8) verbunden ist.

15. Blutzuckermessgerät gemäß der Anspruch 14, wobei das Kommunikationsmodul (7) ein Nahfeldkommunikationschip ist.

16. Blutzuckermessgerät gemäß der Anspruch 14, wobei die Stecheinheit (2) elektrisch mit dem Kommunikationsmodul (7) verbunden ist.

17. Blutzuckermesssystem, das aus einem Blutzuckermessgerät nach einem der Ansprüche 1 bis 7 oder die Blutzuckermessgerät besteht, gemäß einem der Ansprüche 8 bis 16 und ein mobiles Endgerät, wobei das mobile Endgerät so ausgestattet ist, dass es die von der Blutzuckermessgerät gesendete aktuelle Intensität empfängt und die Blutzuckerkonzentration entsprechend der aktuellen Intensität bestimmt.

18. Blutzuckermesssystem gemäß der Anspruch 17, wobei das mobile Endgerät ein Nahfeldkommunikations-Lesegerät umfasst, das verwendet wird, um die vom Blutzuckermessgerät gesendete Stromstärke zu erhalten.

19. Blutzuckermesssystem gemäß der Anspruch 17, wobei das Blutzuckermessgerät ferner eine Temperaturmesseinheit (9) umfasst und das mobile Endgerät zum Empfangen der Temperatur verwendet wird, welche die Temperaturmesseinheit (9) erhält, und wenn sich die Temperatur innerhalb eines voreingestellten Temperaturbereichs befindet, die Blutzuckerkonzentration entsprechend der aktuellen Intensität bestimmt.

## Revendications

1. Le dispositif de mesure de la glycémie, comprenant un socle portable de type carte (1), au moins un autopiqueur (2), dans lequel la lancette (2) est disposée sur le bord du socle portable de type carte (1) et est utilisé pour prélever du sang et générer un courant électrique, et une unité de test (3) disposée sur le socle portable de type carte (1) et électriquement connectée à l'autopiqueur (2) qui est utilisée pour mesurer l'intensité du courant généré et communiquer avec l'extérieur, **caractérisé par le fait que**:
l'autopiqueur (2) comprend une rainure (4) située sur le socle portable de type carte (1) et une unité de mesure de la température (9) est équipée sur le socle portable de type carte (2), la rainure (4) étant recouverte de glucose oxydase ou de glucose déshydrogénase et l'unité de mesure de la température (9) fournissant une certaine plage de température lorsque la glycémie doit être testée.

2. Le dispositif de mesure de la glycémie de la revendication 1, dans lequel le nombre d'autopiqueurs (2) est compris entre 2 et 20.

3. Le dispositif de mesure de la glycémie de la revendication 2, dans lequel ces autopiqueurs (2) sont espacés entre eux et sont disposés le long du bord du socle portable de type carte (1).

4. Le dispositif de mesure de la glycémie de la revendication 1, dans lequel l'autopiqueur (2) est recouvert d'un film protecteur (5), qui est équipé d'une rainure de siphon (6), et l'ouverture de la rainure de siphon (6) est située sur le bord du socle portable de type carte (1) et communique avec l'extérieur.

5. Le dispositif de mesure de la glycémie de la revendication 1, dans lequel l'unité de test (3) comprend un module de communication (7) et un module de mesure de courant (8), et le module de communication (7) est connecté électriquement au module de mesure de courant (8).

6. Le dispositif de mesure de la glycémie de la revendication 5, dans lequel le module de communication (7) est une puce de communication en champ proche.

7. Le dispositif de mesure de la glycémie de la revendication 5, dans lequel l'autopiqueur (2) est connecté électriquement au module de communication (7).

8. Le dispositif de mesure de la glycémie, comprenant un boîtier portable (10) avec une cavité, une lancette (11) placée dans une cavité du boîtier portable (10), au moins un autopiqueur (2), l'autopiqueur (2) étant placé du côté de l'ouverture de la cavité du boîtier portable et servant à prélever du sang et à générer un courant électrique, et une unité de test (3) placée sur le boîtier portable (10) et connectée électriquement à l'autopiqueur (2), servant à détecter l'intensité du courant généré et à communiquer avec l'extérieur, **caractérisé par le fait que**:
l'autopiqueur (2) comprend une rainure (4) prévue sur le boîtier portable (10) et une unité de mesure de la température (9) est équipée sur le socle portable de type carte (2), la rainure (4) étant recouverte de glucose oxydase ou de glucose déshydrogénase et l'unité de mesure de la température (9) devant fournir une certaine plage de température lorsque la glycémie doit être testée.

9. Le dispositif de mesure de la glycémie de la revendication 8, comprenant en outre: une unité de protection de la lancette, la lancette (11) est reliée au boîtier portable (10) par un ressort, et l'unité de protection de la lancette est encastrée dans la cavité du boîtier portable (10), et est placée à l'extrémité avant de la lancette (11)

10. Le dispositif de mesure de la glycémie de la revendication 9, dans lequel l'unité de protection de la lancette comprend une tête de protection (12) et un manchon de protection (13), l'extrémité avant du manchon de protection (13) est pourvue d'un trou de passage et l'extrémité arrière du manchon de protection (13) est encastrée dans la cavité, la lancette (11) peut s'étendre à travers le trou de passage et la tête de protection (12) est utilisée pour bloquer le trou de passage.

11. Le dispositif de mesure de la glycémie de la revendication 8, dans lequel le nombre d'autopiqueurs (2) est compris entre 1 et 4.

12. Le dispositif de mesure de la glycémie de la revendication 9, dans lequel ces autopiqueurs (2) sont espacés entre eux et sont disposés le long de l'ouverture de la cavité du boîtier portable (10).

13. Le dispositif de mesure de la glycémie de la revendication 8, dans lequel l'autopiqueur (2) est recouvert d'un film protecteur (5), qui est équipé d'une rainure de siphon (6), et l'ouverture de la rainure de siphon (6) est située dans l'ouverture de la cavité du boîtier portable (10) et communique avec l'extérieur.

14. Le dispositif de mesure de la glycémie de la revendication 8, dans lequel l'unité de test (3) comprend un module de communication (7) et un module de mesure de courant (8), et le module de communication (7) est connecté électriquement au module de mesure de courant (8).

15. Le dispositif de mesure de la glycémie de la revendication 14, dans lequel le module de communication (7) est une puce de communication en champ proche.

16. Le dispositif de mesure de la glycémie de la revendication 14, dans lequel l'autopiqueur (2) est connecté électriquement au module de communication (7).

17. Système de mesure de la glycémie comprenant le dispositif de mesure de la glycémie selon l'une des revendications 1 à 7 ou le dispositif de mesure de la glycémie selon l'une des revendications 8 à 16 et un terminal mobile, dans lequel le terminal mobile est équipé pour recevoir l'intensité du courant envoyée par le dispositif de mesure de la glycémie et déterminer la concentration de la glycémie en fonction de l'intensité du courant.

18. Le système de mesure de la glycémie de la revendication 17, dans lequel le terminal mobile comprend un dispositif de lecture de la communication en champ proche, qui est utilisé pour obtenir l'intensité du courant envoyé par le dispositif de mesure de la glycémie.

19. Le système de mesure de la glycémie de la revendication 17, dans lequel le dispositif de mesure de la glycémie comprend en outre une unité de mesure de la température (9) et le terminal mobile est utilisé pour recevoir la température que l'unité de mesure de la température (9) obtient, si la température est dans la plage de température prédéfinie, il détermine la concentration de glucose dans le sang en fonction de l'intensité actuelle
